# EUROPEAN PATENT SPECIFICATION

(11) **EP 0 934 528 B1**
(45) Date of publication and mention of the grant of the patent: **04.09.2002**
(21) Application number: 97909212.9
(22) Date of filing: 27.10.1997
(51) Int. Cl.: G01N 33/566, C12Q 1/00

(54) **A METHOD FOR THE IDENTIFICATION OF COMPOUNDS WITH ANXIOLYTIC POTENTIAL**
METHODE ZUR IDENTIFIZIERUNG VON VERBINDUNGEN MIT ANXIOLYTISCHEM POTENTIAL
PROCEDE D'IDENTIFICATION DE COMPOSES A POTENTIEL ANXIOLYTIQUE

(30) Priority: 25.10.1996 DK 118296
(43) Date of publication of application: 11.08.1999
(73) Proprietor: NEUROSEARCH A/S, 2750 Ballerup (DK)
(72) Inventor: JENSEN, Leif, Helth, DK-2600 Glostrup (DK); JOHANSEN, Tina, Holm, DK-2600 Glostrup (DK)
(86) International application number: DK9700475
(87) International publication number: WO98019165

(56) References cited:
- WO-A-93/22681
- WO-A-94/13799
- WO-A-96/33191
- US-A- 5 166 066
- TIPS, Volume 17, May 1996, ERMINIO COSTA et al., "Benzodiazepines on Trial: a Research Strategy for Their Rehabilitation", pages 192-200.
- JOURNAL OF BIOLOGICAL CHEMISTRY, Volume 269, No. 43, 1994, DIETMAR BENKE et al., "Distribution, Prevalence and Drug Binding Profile of Gamma-Aminobutyric Acid Type A Receptor Subtypes Differing in the Beta-Subunit Variant", pages 27100-27107.
- NEUROREPORT, Volume 6, No. 3, February 1995, G. WHITE et al., "Alfa Subunits Influence Zn Block of Gamma2 Containing GABAA Receptor Currents", pages 461-464.
- DIALOG INFORMATION SERVICES, File 154, MEDLINE, Dialog Accession No. 07367310, Medline Accession No. 92383385, GARDNER C.R., "A Review of Recently-Developed Ligands for Neuronal Benzodiazepine Receptors and Their Pharmacological Activities"; & PROG. NEUROPSYCHOPHARMACOL. BIOL. PSYCHIATRY, (ENGLAND), 1992, 16(6), p. 755-81.
- THE JOURNAL OF NEUROSCIENCE, Volume 12, No. 3, March 1992, W. WISDEN et al., "The Distribution of 13 GABAA Receptor Subunit mRNAs in the Rat Brain. I. Telencephalon, Diencephalon, Mesencephalon", pages 1040-1062.
- THE JOURNAL OF NEUROSCIENCE, Volume 15, No. 10, October 1995, HARTMUT LUEDDENS et al., "GABA Antagonists Differentiate Between Recombinant GABAA/Benzodiazepine Receptor Subtypes", pages 6957-6962.

## Description

### TECHNICAL FIELD

The present invention relates to the use of a cloned GABA_{A} receptor subtype composed of the α₂, β₃ and γ₂ subunits for the identification of benzodiazepine receptor ligands with selective anxiolytic properties (i.e. non-sedative anxiolytica).

### BACKGROUND ART

Receptors for the major inhibitory neurotransmitter, γ-aminobutyric acid (GABA), are divided into two main classes, GABA_{A} receptors and GABA_{B} receptors.

GABA_{A} receptors are members of a ligand-gated ion channel family, and are the most abundant Inhibitory receptors in the mammalian brain. Each GABA_{A} receptor complex comprises a chloride ion channel that controls the chloride flux across the neuronal membrane, along with multiple recognition sites for small modulatory molecules such as benzodiazepines, barbiturates, picrotoxin, and certain steroids. When GAGA interacts with its receptor, the ion channel is opened, the chloride flux is enhanced, and the cell becomes less responsive to excitatory stimuli. This GABA induced ion current can be regulated by various agents, including agents that interact with the benzodiazepine receptor or recognition site.

Agents that bind or interact with the modulatory sites on the GABA_{A} receptor complex, e.g. the benzodiazepine receptor, and have a positive modulatory effect on the action of GABA, are called benzodiazepine receptor agonists or partial agonists. Agonists generally produce muscle relaxant, hypnotic, sedative, anxiolytic, and/or anticonvulsant effects.

Benzodiazepine receptor ligands with negative modulatory effect on the action of GABA are termed inverse agonists, while benzodiazepine receptor ligands with no intrinsic activity are termed antagonists.

Numerous compounds belonging to different series of compounds having affinity for the benzodiazepine receptors have been synthesized during the last three decades. In particular it has been the aim of several groups to develop benzodiazepine receptor modulators having potent anxiolytic activity and devoid of sedative effects. However, although the benzodiazepine receptor sites are still considered as very attractive biological sites for interfering with the CNS to treat various disorders and diseases, then nearly all previously synthesized compounds acting at these receptor sites have failed during clinical development because of unacceptable side effects.

The GABA_{A} receptors are structurally constituted macromolecular heteropentameric assemblies, containing a combinations of α, β, and γ/δ protein subunits. Several subunits of such GABA_{A} receptors (α₁₋₆, β₁₋₃, δ and γ₁₋₃) have been characterized using techniques of modern molecular biology. Considering the number of individual mammalian GABA_{A} receptor subunits, the number of pentameric subtypes with different subunit permutations that theoretically could exist, is overwhelming. It has therefore been a major challenge to delineate the subunit complements of native GABA receptors and to resolve their physiological role.

It is believed that specific GABA_{A} receptor subtypes are responsible for mediating the anxiolytic effect of benzodiazepines. But the specific subtypes and/or subunits involved in the patophysiology of anxiety disorders Is not known at present.

From anatomical/behavioral studies in rats it has been shown that the anti-conflict effect of the benzodiazepine Midazolam™ Is mediated at the baso-lateral amygdaloid nucleus (European Journal of Pharmacology, 1982 **82** 115-116, and Neuroscience Letters, 1985 **53** 285-288). As mentioned above six α subunits exists and benzodiazepines are believed to bind to α-subunits or at the interface between the α subunit and the β subunit. The α₁ subunit is distributed more or less uniformly throughout the rat brain, and is believed to be involved in the sedative affects of benzodiazepine receptor ligands. The affinity of benzodiazepines for α₄ and α₆ subunits is low and it is therefore highly unlikely that benzodiazepines mediate their effects via these subunits.

From anatomical studies with in situ hybridization it has been shown that α₁, α₂, α₃, α₄ and α₅ subunits are present in the baso-lateral amygdaloid nucleus, α₂ being the most abundant α-subunit (The Journal of Neuroscience, 1992 **12** (3) 1040-1062).

Additionally, It has been shown that the β₃ subunit is the most abundant β subunit in the baso-lateral amygdaloid nucleus and that the β₃ subunit seems predominantly to be expressed in combination with α₂ subunits (The Journal of Neuroscience, 1992 **12** (3) 1040-1062, and Biochem. J. 1995 **310** 1-9). Likewise the most abundant γ subunit in the baso-lateral amygdaloid nucleus is the γ₂ subunit.

These findings suggests that the α₂β₃γ₂ GABA_{A} receptor subtype may be the sole mediator of the anxiolytic effect of benzodiazepines.

WO 94/13799 describes a stably transfected cell line expressing different subtypes of the GABA_{A}-receptors.

TIPS, 1996 **17** 192-200 describes the anxiolytic potential of the benzodiazepine and non-benzodiazepine compounds known In the art for having anxiolytic potential but also having severe side-effects such as ataxia, sedation, amnesia, etc.

Journal of biological chemistry, 1994 **269** (43) 27100-27107 describes the distribution, prevalence and drug binding profile of some GABA receptor subtypes.

WO 93/22681 describes a method for drug screening wherein a competitive reaction between a radiolabelled compound and a test compound is measured.

Progress in Neuro-Psychopharmacology & Biological Psychiatry, 1992 **16** (6) 755-781 mentions some α subtypes but does not combine with other subtypes of the GABA-receptor complex.

Finally, WO 96/33191 discloses benzimidazole compounds that modulate the activity of the GABA_{A}-receptor complex.

By measuring the affinity and efficacy of a novel benzodiazepine receptor agonist having potent anxiolytic properties and low sedative effects on cloned GABA_{A} receptor subtypes it was found that this novel benzodiazepine receptor ligand is a full agonist to the α₂β₃γ₂ GABA_{A} receptor subtype and a partial agonist the α₁β₂γ₂ GABA_{A} receptor subtype.

Based on these findings a novel method for the identification of compounds with anxiolytic potential has been developed.

### SUMMARY OF THE INVENTION

It is an object of the present invention to provide a method for the identification of benzodiazepine receptor ligands with selective anxiolytic properties (i.e. non-sedative) by measuring the affinity and/or efficacy of the test compounds to a test compound to other GABA_{A} receptor subtypes which are known not to mediate selective anxiolytic effects. The present invention provides a valuable tool for screening chemical libraries for compounds with selective anxiolytic activity and for designing drugs with selective anxiolytic activity.

Accordingly, in its first aspect, the invention provides a method for the identification of chemical compounds having anxiolytic potential and no or only low sedative effects, which method comprises the steps of
(i) measuring the affinity and/or efficacy of a chemical compound at a cloned α₂β₃γ₂ GABA_{A} receptor;
(ii) comparing the affinity and/or efficacy measured in step (i) with the affinity and/or efficacy of the same chemical compound at a different cloned GABA_{A} receptor subtype; and
(iii) selecting chemical compounds which are selective benzodiazepine receptor agonists at the α₂β₃γ₂ GABA_{A} receptor subtype.

### DETAILED DISCLOSURE OF THE INVENTION

The present invention provides for the identification of chemical compounds having potential as benzodiazepine receptor ligands with anxiolytic activity and no or only low sedative effects.

The method of the invention comprises measuring the affinity and/or efficacy of a chemical compound (the test compound) for binding onto a cloned α₂β₃γ₂ GABA_{A} receptor, and comparing the affinity and/or efficacy so determined with the affinity and/or efficacy of the same compound for binding onto another cloned GABA_{A} receptor subtype. In this way compounds which are selective benzodiazepine receptor agonists at the α₂β₃γ₂ GABA_{A} receptor subtype can be identified.

As defined herein, selective affinity for a GABA_{A} receptor means that the affinity of a compound for the receptor is higher than the affinity of the compound to other GABA_{A} receptors.

Also, as defined herein, a selective agonist of a GABA_{A} receptor is a positive modulator of a GABA_{A} receptor which have a higher efficacy to this receptor that to other GABA_{A} receptors.

In a preferred embodiment, the method of the invention comprises
(i) measuring the affinity of test compounds to cloned α₂β₃γ₂ GABA_{A} receptors and to other cloned GABA_{A} receptor subtypes;
(ii) selecting compounds with selective affinity for the cloned α₂β₃γ₂ GABA_{A} receptors;
(iii) measuring the efficacy of the selected compounds at the cloned α₂β₃γ₂ GABA_{A} receptor; and
(iv) selecting compounds which are selective agonist at the cloned GABA_{A} receptor.

In another preferred embodiment the method of the invention comprises
(i) measuring the affinity of test compounds to a cloned α₂β₃γ₂ GABA_{A} receptor and to other cloned GABA_{A} receptor subtypes;
(ii) selecting compounds with affinity for the cloned α₂β₃γ₂ GABA_{A} receptor;
(iii) measuring the efficacy of the selected compounds at the cloned GABA_{A} receptors; and
(iv) selecting compounds which are selective agonists at the cloned α₂β₃γ₂ GABA_{A} receptor.

In the method of the invention, the affinity of the test compounds for the GABA_{A} receptor subtypes may be determined by the ability of the test compounds to displace a radio labeled non-selective, high affinity benzodiazepine receptor ligand, such as tritium isotope radio labeled Flumazenil™ or Flunitrazepam™.

Alternatively, the efficacy of the test compounds for the GABA_{A} receptor subtypes may be determined by measuring the affinity of the compounds for the receptor in presence an in absence of GABA and calculating the GABA-ratio.

In yet another embodiment, the efficacy of the test compounds for the GABA_{A} receptor may be determined by measuring the chloride flux using patch clamp technique.

In another preferred embodiment, the additional cloned GABA_{A} receptor subtypes employed in the method of the invention, andnot being a cloned α₂β₃γ₂ GABA_{A} receptor, are subtypes which are known not to mediate selective anxiolytic effects. More preferred this additional cloned GABA_{A} receptor subtypes contain an α₁, α₃ or α₅ GABA_{A} receptor subunit. In a most preferred embodiment, this additional cloned GABA_{A} receptor subtypes contain the subunit combination α₁β₃γ₂ or α₃β₂γ₂.

In a particular embodiment of the invention, stabile CHO (Chinese hamster ovary) cells expressing cloned rat or human GABA receptor subunits is used to screen test compounds for their affinity to the α₂β₃γ₂ GABA_{A} receptor subtype. The affinity of the test compounds to this receptor subtype is compared with the affinity of the compounds to GABA_{A} receptor types which are believed not to mediate selective anxiolytic effects, e.g. GABA_{A} receptor subtypes containing an α₁ receptor subunit. The affinity of the test compounds for the cloned receptor is determined by measuring the ability of the compounds to displace a non-selective radio labeled benzodiazepine receptor ligand, such as tritium isotope radio labeled Flumazenil™ or Flunitrazepam™. Inhibition of binding is measured by a conventional in vitro binding assays using membrane preparations of cells expressing cloned GABA_{A} receptor subtypes.

The efficacy of the test compounds, i.e. whether the compounds are agonists, Inverse agonists or antagonists at the benzodiazepine receptor, can also be determined by calculating the GABA-ratio of the compounds for each of the receptor subtypes employed. GABA-ratio is the ratio between the affinity of a test compound measured in presence of GABA and the affinity of the compound measured in absence of GABA. Benzodiazepine receptor ligands which are full agonists generally have a GABA ratio between 1.8-3, partial agonists have a ratio between 1.0-1.8, antagonists have a ratio around 1, whereas inverse agonist have a GABA ratio around 0.5 -1. Calculation of GABA-ratio therefore provides an indication of the efficacy of the test compound.

The efficacy of the compound can also be measured in electrophyslological experiments using patch clamp technique.

In the method of the invention any cell expressing GABA_{A} receptor subunits may be employed. Preferred cells are CHO cells, or other rodent fibroblast cell lines such as BHK (baby kidney hamster cells) and mouse Ltk⁻ cells, insects cells, such as Sf-9 cells, and HEK293 or HeLa cells. The GABA_{A} subunits expressed by the cells may be of animal (rat or bovine) or human origin.

The chemical compound identified by the method of the invention may be used as a medicament and in pharmaceutical compositions.

The chemical compound identified by the method of the invention may be used in a method for the treatment of an individual suffering from anxiety comprising administering to said individual a therapeutically effective amount of a compound which is a selective benzodiazepine receptor agonist at the α₂β₃γ₂ GABA receptor.

### EXAMPLES

The invention is further illustrated with reference to the following example which is not intended to be in any way limiting to the scope of the invention as claimed.

The affinity of 5-acetyl-1-(3-(3-pyridyl)phenyl)benzimidazole O-ethyl oxime for various GABA_{A} receptor subtypes was studied using recombinant GABA_{A} receptors expressed in insect cells. The ratio between affinity for the compound in the absence and presence of GABA (GABA ratio) depended on the subunit combination of the cloned receptors. The compound had a GABA ratio corresponding to a full agonist at the α₂β₃γ₂ GABA_{A} receptor subtype and a GABA ration corresponding to a partial agonist at the α₁β₂γ₂ GABA_{A} receptor subtype.

## Claims

1. A method for the identification of a chemical compound having anxiolytic potential and no or only low sedative effects, which method comprises the steps of
(i) measuring the affinity and/or efficacy of a chemical compound at cloned α₂β₃γ₂ GABA_{A} receptors;
(ii) comparing the affinity and/or efficacy measured in step (i) with the affinity and/or efficacy of the same chemical compound at different cloned GABA_{A} receptor subtypes; and
(iii) selecting the chemical compound that is a selective benzodiazepine receptor agonist at the α₂β₃γ₂ GABA_{A} receptor subtypes.

2. The method according to claim 1, comprising
(i) measuring the affinity of the test compound to cloned α₂β₃γ₂ GABA_{A} receptors and to different cloned GABA_{A} receptor subtypes;
(ii) selecting the compound which has selective affinity for the cloned α₂β₃γ₂ GABA_{A} receptors;
(iii) measuring the efficacy of the selected compound at the cloned α₂β₃γ₂ GABA_{A} receptors; and
(iv) selecting the compound which is a selective agonist at the cloned GABA_{A} receptors.

3. The method according to claim 1, comprising
(i) measuring the affinity of the test compound to cloned α₂β₃γ₂ GABA_{A} receptors and to different cloned GABA_{A} receptor subtypes;
(ii) selecting the compound which has affinity for the cloned α₂β₃γ₂ GABA_{A} receptors;
(iii) measuring the efficacy of the selected compound at the cloned GABA_{A} receptors; and
(iv) selecting the compound which is a selective agonist at the cloned α₂β₃γ₂ GABA_{A} receptors.

4. The method according to any of claims 1-3, wherein the affinity of the test compound for the GABA_{A} receptor subtypes is determined by measuring the ability of the test compound to displace a radio labeled non-selective, high affinity benzodiazepine receptor ligand, such as tritium isotope radio labeled Flumazenil™ or Flunitrazepam™.

5. The method according to any of claims 1-4, wherein the efficacy of the test compound for the GABA_{A} receptor subtypes is determined by measuring the affinity of the compound for the receptors in the presence and in the absence of GABA, and calculating the GABA-ratio.

6. The method according to any of claims 1-4, wherein the efficacy of the test compound for the GABA_{A} receptor subtypes is determined by measuring the chloride flux using patch clamp technique.

7. The method according to any of claims 1-6, wherein the cloned GABA_{A} receptor subtypes that is different from the cloned α₂β₃γ₂ GABA_{A} receptors, are subtypes which are known not to mediate selective anxiolytic effects.

8. The method according to claim 1, wherein the cloned GABA_{A} receptor subtypes that is different from the cloned α₂β₃γ₂ GABA_{A} receptors, contain the α₁, α₃ or α₅ GABA_{A} receptor subunits.

9. The method according to claim 1, wherein the cloned GABA_{A} receptor subtypes that is different from the cloned α₂β₃γ₂ GABA_{A} receptors, contain the subunit combination α₁β₃γ₂ or α₃β₂γ₂.

## Patentansprüche

1. Ein Verfahren zur Identifizierung einer chemischen Verbindung, die anxiolytisches Potential aber keine oder nur geringe sedative Wirkungen aufweist, wobei das Verfahren die Schritte umfasst:
(i) Messung der Affinität und/oder Wirksamkeit einer chemischen Verbindung für die klonierten α₂β₃γ₂-GABA_{A}-Rezeptoren;
(ii) Vergleichen der in Schritt (i) gemessenen Affinität und/oder Wirksamkeit mit der Affinität und/oder Wirksamkeit der gleichen chemischen Verbindung für die verschiedenen klonierten GABA_{A}-Rezeptorsubtypen; und
(iii) Auswählen der chemischen Verbindung, die ein selektiver Benzodiazepinrezeptoragonist für die α₂β₃γ₂-GABA_{A}-Rezeptorsubtypen ist.

2. Das Verfahren gemäß Anspruch 1, umfassend
(i) Messung der Affinität der Versuchsverbindung zu klonierten α₂β₃γ₂-GABA_{A}-Rezeptoren und zu verschiedenen klonierten GABA_{A}-Rezeptorsubtypen;
(ii) Auswählen der Verbindung, die eine selektive Affinität für die klonierten α₂β₃γ₂-GABA_{A}-Rezeptoren aufweist;
(iii) Messung der Wirksamkeit der ausgewählten Verbindung für die klonierten α₂β₃γ₂-GABA_{A}-Rezeptoren; und
(iv) Auswählen der Verbindung, welche ein selektiver Agonist für die klonierten GABA_{A}-Rezeptoren ist.

3. Das Verfahren gemäß Anspruch 1, umfassend
(i) Messung der Affinität der Versuchsverbindung zu klonierten α₂β₃γ₂-GABA_{A}-Rezeptoren und zu verschiedenen klonierten GABA_{A}-Rezeptorsubtypen;
(ii) Auswählen der Verbindung, die eine Affinität zu den klonierten α₂β₃γ₂-GABA_{A}-Rezeptoren aufweist;
(iii) Messung der Wirksamkeit der ausgewählten Verbindung an den klonierten GABA_{A}-Rezeptoren; und
(iv) Auswählen der Verbindung, die ein selektiver Agonist für die klonierten α₂β₃γ₂-GABA_{A}-Rezeptoren ist.

4. Das Verfahren gemäß einem der Ansprüche 1 bis 3, bei dem die Affinität der Versuchsverbindung für die GABA_{A}-Rezeptorsubtypen durch die Messung der Fähigkeit der Versuchsverbindungen bestimmt wird, radiomarkierte nicht-selektive, hochaffine Benzodiazepinrezeptorliganden, wie Tritiumisotop radiomarkiertes Flumazenil™ oder Flunitrazepam™, zu verdrängen.

5. Das Verfahren gemäß einem der Ansprüche 1 bis 4, worin die Wirksamkeit der Versuchssubstanz für die GABA_{A}-Rezeptorsubtypen durch Messung der Affinität der Verbindung zu den Rezeptoren in der Anwesenheit und in der Abwesenheit von GABA bestimmt wird und das GABA-Verhältnis berechnet wird.

6. Das Verfahren gemäß einem der Ansprüche 1 bis 4, worin die Wirksamkeit der Versuchsverbindung für die GABA_{A}-Rezeptorsubtypen durch das Messen des Chloridstromes unter Verwendung der Patch-clamp-Technik bestimmt wird.

7. Das Verfahren gemäß einem der Ansprüche 1 bis 6, worin die klonierten GABA_{A}-Rezeptorsubtypen, die von den klonierten α₂β₃γ₂-GABA_{A}-Rezeptoren unterschiedlich sind, Subtypen sind, von denen bekannt ist, dass sie nicht selektive anxiolytische Effekte vermitteln.

8. Das Verfahren gemäß Anspruch 1, worin die klonierten GABA_{A}-Rezeptorsubtypen, die von den klonierten α₂β₃γ₂-GABA_{A}-Rezeptoren verschieden sind, die α₁-, α₃oder α₅-Rezeptoruntereinheiten enthalten.

9. Das Verfahren gemäß Anspruch 1, worin die klonierten GABA_{A}-Rezeptorsubtypen, die von den klonierten α₂β₃γ₂-GABA_{A}-Rezeptoren verschieden sind, die Kombination der Untereinheiten α₁β₃γ₂ oder α₃β₂γ₂ enthalten.

## Revendications

1. Procédé d'identification d'un composé chimique possédant un potentiel anxiolytique et pas ou seulement peu d'effets sédatifs, ce procédé comprenant les étapes de :
(i) mesure de l'affinité et/ou l'efficacité d'un composé chimique à des récepteurs α₂β₃γ₂ GABA_{A} clones ;
(ii) comparaison de l'affinité et/ou l'efficacité mesurée dans l'étape (i) à l'affinité et/ou l'efficacité du même composé chimique à des sous-types différents de récepteurs GABA_{A} clonés ; et
(iii) choix du composé chimique qui est un agoniste sélectif des récepteurs de la benzodiazépine aux sous-types α₂β₃γ₂ de récepteurs GABA_{A}.

2. Procédé selon la revendication 1, comprenant :
(i) une mesure de l'affinité du composé d'essai avec des récepteurs α₂β₃γ₂ GABA_{A} clonés et avec des sous-types différents de récepteurs GABA_{A} clonés ;
(ii) un choix du composé qui possède une affinité sélective pour les récepteurs α₂β₃γ₂ GABA_{A} clonés ;
(iii) une mesure de l'efficacité du composé choisi aux récepteurs α₂β₃γ₂ GABA_{A} clonés ; et
(iv) un choix du composé qui est un agoniste sélectif aux récepteurs GABA_{A} clonés.

3. Procédé selon la revendication 1, comprenant :
(i) une mesure de l'affinité du composé d'essai avec des récepteurs α₂β₃γ₂ GABA_{A} clonés et avec des sous-types différents de récepteurs GABA_{A} clonés ;
(ii) un choix du composé qui possède une affinité pour les récepteurs α₂β₃γ₂ GABA_{A} clonés ;
(iii) une mesure de l'efficacité du composé choisi aux récepteurs GABA_{A} clonés ; et
(iv) un choix du composé qui est un agoniste sélectif aux récepteurs α₂β₃γ₂ GABA_{A} clonés.

4. Procédé selon l'une quelconque des revendications 1 à 3, dans lequel l'affinité du composé d'essai pour les sous-types de récepteurs GABA_{A} est déterminée en mesurant l'aptitude du composé d'essai à déplacer un ligand des récepteurs de la benzodiazépine non-sélectif, de forte affinité et à marquage radioactif, tel que le Flumazénil (marque de commerce) ou le Flunitrazépam (marque de commerce) marqué radioactivement à l'isotope de tritium.

5. Procédé selon l'une quelconque des revendications 1 à 4, dans lequel l'efficacité du composé d'essai pour les sous-types de récepteurs GABA_{A} est déterminée en mesurant l'affinité du composé pour les récepteurs en présence et en l'absence de GABA, et en calculant le taux de GABA.

6. Procédé selon l'une quelconque des revendications 1 à 4, dans lequel l'efficacité du composé d'essai pour les sous-types de récepteurs GABA_{A} est déterminée en mesurant le flux de chlorure en utilisant la technique de suture de fragment.

7. Procédé selon l'une quelconque des revendications 1 à 6, dans lequel les sous-types de récepteurs GABA_{A} clonés qui sont différents des récepteurs α₂β₃γ₂ GABA_{A} clonés sont des sous-types qui sont connus pour de pas favoriser d'effets anxiolytiques sélectifs.

8. Procédé selon la revendication 1, dans lequel les sous-types de récepteurs GABA_{A} clonés qui sont différents des récepteurs α₂β₃γ₂ GABA_{A} clonés contiennent les sous-unités α₁, α₃ ou α₅ de récepteurs GABA_{A}.

9. Procédé selon la revendication 1, dans lequel les sous-types de récepteurs GABA_{A} clonés qui sont différents des récepteurs α₂β₃γ₂ GABA_{A} clonés contiennent la combinaison de sous-unités α₁β₃γ₂ ou α₃β₂γ₂.
